Europäisches Patentamt

European Patent Office

Office européen des brevets

(19)

(11) Publication number: **0 380 084**
**A2**

(12) **EUROPEAN PATENT APPLICATION**

(21) Application number: **90101430.8**

(22) Date of filing: **24.01.90**

(51) Int. Cl.5: **A61K 31/70, A61K 39/395, A61K 37/02**

(30) Priority: **27.01.89 US 303698**

(43) Date of publication of application:
**01.08.90 Bulletin 90/31**

(84) Designated Contracting States:
**AT BE CH DE DK ES FR GB GR IT LI LU NL SE**

(71) Applicant: **The Biomembrane Institute**
**201 Elliott Avenue West Suite 305**
**Seattle Washington 98119(US)**

(72) Inventor: **Hakomori, Sen-itiroh**
**2024 80th S.E.**
**Mercer Island, Washington 98040(US)**
Inventor: **Eggens, Ivan**
**Ornbacken 254**
**126 51 Hagersten, Stockholm(SE)**
Inventor: **Fenderson, Bruce A.**
**4224 N.E.110th**
**Seattle, Washington 98125(US)**
Inventor: **Toyokuni, Tatsushi**
**3716 N.E. 75th Apt. 404**
**Seattle, Washington 98115(US)**

(74) Representative: **Brown, John David et al**
**FORRESTER & BOEHMERT**
**Widenmayerstrasse 4/I**
**D-8000 München 22(DE)**

(54) **Method for the inhibition of cell-cell and cell substratum interactions by the blocking of carbohydrate-carbohydrate interactions.**

(57) Methods for the inhibition of cell-cell and cell-substratum interactions by the blocking of carbohydrate-carbohydrate interactions are disclosed. Inhibition of cell-cell and cell-substratum interactions by the methods of the present invention permit the inhibition of a variety of biological phenomena, including metastases, mammalian diseases that are microbial in origin, mammalian fertilization, and plant pathologies. Suitable agents for blocking carbohydrate-carbohydrate interactions include saccharides, glycoconjugates, and antibodies directed against carbohydrate determinants.

# METHODS FOR THE INHIBITION OF CELL-CELL AND CELL-SUBSTRATUM INTERACTIONS BY THE BLOCKING OF CARBOHYDRATE-CARBOHYDRATE INTERACTIONS

## Technical Field

The present invention is generally directed toward the inhibition of cell-cell and cell-substratum interactions by the blocking of carbohydrate-carbohydrate interactions, and more specifically, to the inhibition of metastases, mammalian diseases that are microbial in origin, mammalian fertilization, and plant pathologies.

## Background of the Invention

Cell-cell and cell-substratum (e.g., basement membrane) interactions are essential to a wide variety of biological processes. Despite the level of attention that this topic has received in current studies, the biochemical mechanism of cell-cell interaction and adhesion is not well understood. Although pericellular adhesive proteins (e.g., fibronectin, laminin, thrombospondin, N-CAM, E-cadherin) and their cellular receptors may play an important role in nonspecific cellular adhesion, these molecules do not appear to be specific cell recognition molecules. While specific carbohydrate chains at a cell's surface can provide the necessary specificity for the initial step of specific cell recognition, the identity of the molecules that recognize carbohydrates at the counterpart cell has been unclear.

The basic model of the biochemical mechanisms of cell-cell interaction, as proposed by Tyler (Growth 10:7-19, 1947) and by Weiss (Yale J. Biol. Med. 19:235-278, 1947), is based on the assumption that cells present complementary "lock and key" structures similar to antigen- or antibody-like molecules expressed at the cell surface. This model can be extended to specific cell surface carbohydrate determinants and their recognition proteins, i.e., lectins, glycosyltransferases, and glycosyl hydrolases. The presence of carbohydrate binding proteins (lectins) at the animal cell surface, and their physiological and cell biological implications, have been discussed frequently. However, cell surface localization and function appear to be limited to certain types of cells, i.e., hepatocytes, macrophages, and tumor cells. Furthermore, the majority of animal cell lectins are located in the cytoplasm or nucleus, and their function may not always be "ektobiological"; the majority of their specificities are limited to galactosyl, lactosyl or lactosaminyl structures. Consequently, it is difficult to reconcile this model with the highly specific nature of cell interactions, particularly the recognition between identical types of cells, which provides the basis for sorting one homotypic population out of a heterotypic assemblage of cells.

One area in which cell-cell and cell-substratum interactions play an essential role is that of the spread of cancer by metastasis colony formation. These interactions are important to attachment of a malignant tumor cell to normal tissue and recognition between tumor cells. Current cancer therapies are able to cure only about fifty percent of patients who develop a malignant tumor. In most human malignancies, metastases are the major cause of death. One reason for this is that distant metastases are often too small to be detected at the time the primary tumor is treated. Furthermore, widespread initiation of metastatic colonies usually occurs before clinical symptoms of metastatic disease are evident. The size and age variation in metastases, their dispersed anatomical location, and their heterogeneous composition are all factors that hinder surgical removal and limit the concentration of anticancer drugs that can be delivered to the metastatic colonies. Consequently, there is a need for effective inhibition of metastases.

Another area in which cell-cell and cell-substratum interactions play an essential role is that of mammalian diseases that are microbial, e.g., bacterial or viral, in origin. These interactions are important in attachment of microbes to a mammalian host (See, e.g., "Adhesion and Microorganism Pathogenicity," Proceedings of CIBA Foundation Symposium, C. Elliott et al., eds., Pitman Medical, Bath, England, 1981) and recognition between microbes. Despite defensive measures, such as physical barriers utilized by the mammalian host, microorganisms are frequently able to invade, colonize, and thus cause disease in the host. For many individuals, such as those receiving chemotherapy, it is imperative that they are kept free of infections. Frequently, antibiotics are administered prophylactically. However, chronic use of subtherapeutic or therapeutic dosages of antibiotics increases the risk of selection for antibiotic-resistant microorganisms. Consequently, there is a need for a way to prevent bacterial infections without compromising the therapeutically useful antibiotics.

Another area in which cell-cell or cell-substratum interactions play an essential role is that of fertilization. These interactions are important in attachment of sperm to eggs. A variety of compositions and devices have been used in an effort to inhibit fertilization. During the decade 1973-1982 (the most recent period for which extensive data are available), the use of oral contraceptives among women in the United States sharply declined (Bachrach, Fam. Plann. Perspect. 16:253-259, 1984). This may be traced, at least in part, to complications associated with the use of oral contraceptives, including an increased risk of cancer, blood clots, and associated disorders. Use of "the pill" has therefore been supplanted by the use of less effective barrier methods, such as condoms, diaphragms, and foams, and by sterilization. Another alternative, the intrauterine device (IUD), has also been associated with health risks to the user. Many of these devices have been withdrawn from the marketplace. The traditional barrier methods often rely in part on spermicidal agents for their effectiveness. In general, only a limited number of sperm-inhibiting compounds have proven to be acceptable as contraceptive agents. The most widely used spermicidal agents are harsh, cytotoxic chemicals which may cause irritation or allergic reactions in users. There is thus a need for reversible, dependable methods of contraception which are not associated with health risks to the user or her offspring.

Cell-cell and cell-substratum interactions are also important in plant pathologies. These interactions are important in attachment of a plant pathogen to a plant and recognition between plant pathogens. Detrimental pathogens, such as Agrobacterium species and members of the Pseudomonas syringae group, infect and transform plants. Despite the existence of plant defense mechanisms, diseases such as crown gall tumors and "hairy roots" (elicited by virulent strains of Agrobacterium tumefacienas and Agrobacterium rhizogenes, respectively) occur. These and other plant diseases have had a significant economic impact. Thus, there is a need to manipulate plant-microbe interactions for the improvement of plant crop production and quality.

Due to the essential role that cell-cell and cell-substratum interactions play in a number of biological processes, there is a need in the art for methods of inhibiting these interactions in situations where these interactions are not desirable. The present invention fills this need and further provides other related advantages.

Summary of the Invention

Briefly stated, the present invention provides a variety of methods for inhibiting cell-cell or cell-substratum interactions. In one aspect of the present invention, a method for inhibiting cell-cell or cell-substratum interactions within a biological preparation is provided. The method comprises incubating the biological preparation with an agent that blocks carbohydrate-carbohydrate interactions, thereby inhibiting cell-cell or cell-substratum interactions.

A method for inhibiting cell-cell or cell-substratum interactions in a warm-blooded animal is also provided. The method comprises administering to a warm-blooded animal an effective amount of an agent that blocks carbohydrate-carbohydrate interactions, thereby inhibiting cell-cell or cell-substratum interactions.

Another aspect of the present invention provides a method for inhibiting metastases in a warm-blooded animal. The method comprises administering to a warm-blooded animal a therapeutically effective amount of an agent that blocks carbohydrate-carbohydrate interactions, wherein at least one member of the carbohydrate-carbohydrate interaction resides on a malignant tumor cell.

The present invention is also directed toward a method of inhibiting microbial infections in a warm-blooded animal. The method comprises administering to a warm-blooded animal a therapeutically effective amount of an agent that blocks carbohydrate-carbohydrate interactions, wherein at least one member of the carbohydrate-carbohydrate interaction resides on a microbe. Within a related aspect, a method for inhibiting dental plaque in a warm-blooded animal is provided. The method comprises administering to a warm-blooded animal an effective amount of an agent that blocks carbohydrate-carbohydrate interactions, wherein at least one member of the carbohydrate-carbohydrate interaction resides on an oral bacterial cell.

Another aspect of the present invention provides as method for inhibiting fertilization in a warm-blooded animal. The method comprises administering to a warm-blooded animal an effective amount of a composition comprising an agent that blocks carbohydrate-carbohydrate interactions between sperm and egg, and a physiologically acceptable carrier or diluent.

Within a related aspect of the present invention, a method for inhibiting plant-plant pathogen interactions is provided. The method comprises administering to the plant an effective amount of an agent that blocks carbohydrate-carbohydrate interactions, wherein at least one member of the carbohydrate-carbohydrate interaction resides on a plant pathogen.

These and other aspects of the present invention will become evident upon reference to the following detailed description and attached drawings.

Brief Description of the Drawings

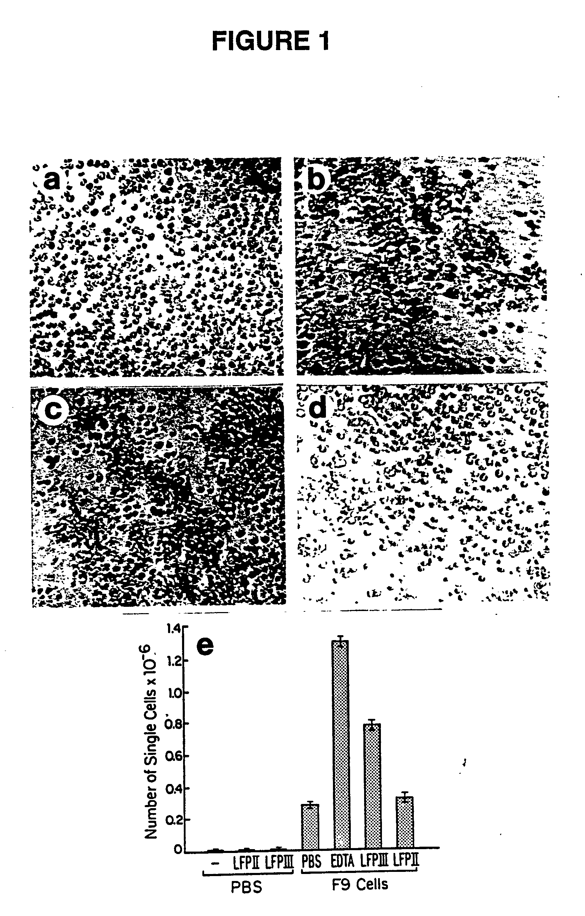

Figure 1 (panels a, b, c, d, and e) provides microphotographs of F9 cell aggregation and the effect of 2 mM LFP II and III. Panel a: control cells suspension in the presence of 50 mM EDTA, in which aggregation did not occur. Panel b: aggregation of F9 cells in the presence of PBS containing 0.9 mM $CaCl_2$ and 0.5 mM $MgSO_4$. Panel c: aggregation of F9 cells in the presence of 2 mM LFP II. Panel d: F9 cells in the presence of 2 mM LFP III, in which aggregation was inhibited. Panel e: semiquantitative analysis of inhibition of F9 cell aggregation by 50 mM EDTA, 2 mM LFP III, and 2 mM LFP II, respectively. The degree of aggregation was determined by counting the number of single, nonaggregated cells (ordinate) using a Coulter Counter under defined conditions. Blank counting values for medium without cells is marked "PBS" in the absence (-) and presence of LFP II and LFP III, as shown in the abscissa of panel e. F9 cells incubated in PBS containing bivalent cation (as in panel b) are marked as "F9 cells PBS" in panel e. Cells in 50 mM EDTA (as in panel a) are marked as "EDTA F9 cells," and those in the presence of LFP II (as in panel c) and LFP III (as in panel d) are marked, respectively, "LFP II F9 cells" and "LFP III F9 cells" in panel e. Values represent means of triplicate determinations; vertical bars represent standard deviations.

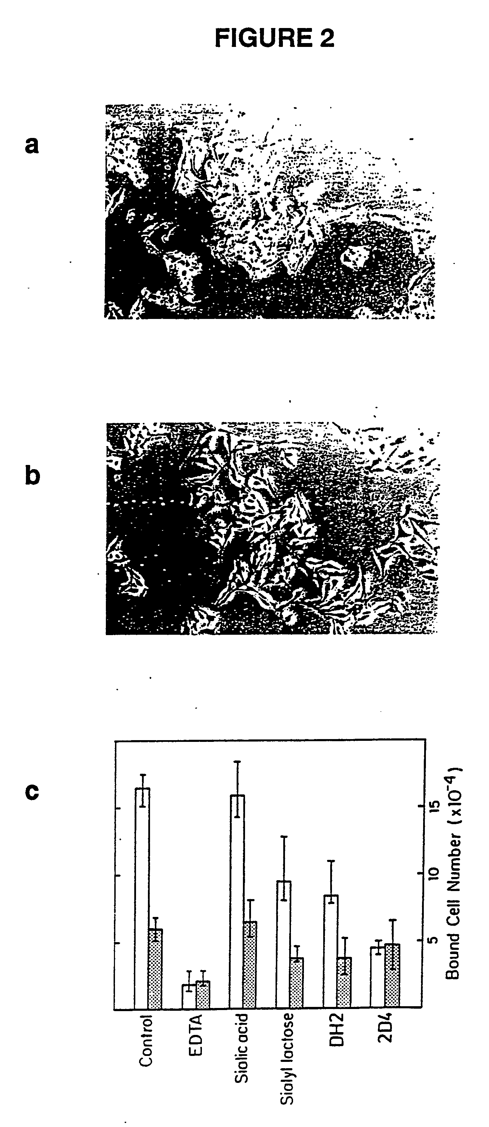

Figure 2 (panels a, b and c) provides microphotographs of adhesion of mouse lymphoma L5178 AA12 cell line on B16 melanoma cells, and the effect of various reagents on this adhesion. Panel a: L5178 AA12 lymphoma cells adherent on B16 melanoma. Lymphoma clone AA12 cells (expressing Gg3) were co-cultured on B16 melanoma (spindle shape) for 24 hrs. Medium was then removed and cells were washed twice with serum-free RPMI 1640. AA12 lymphoma cells adherent on B16 melonoma resisted washing. Panel b: L5178 lymphoma clone AV27 cells (not expressing Gg3) were incubated, but were subsequently washed out. Panel c: effect of EDTA, sialic acid, sialyllactose, anti-GM$_3$ antibody DH2, and anti-Gg3 MAb 2D4 on interaction of AA12 or AV27 cells with B16 cells. The degree of adhesion of lymphoma cells to melanoma cells was determined by measuring the $^3H$ remaining after incubating B16 cells with $^3H$-labeled AA12 or $^3H$-labeled AV27 cells and washing with serum-free fresh medium. Open and shaded columns show adhesion of AA12 and AV27 cells respectively. Effects of (respectively) 10 mM EDTA, 5 mM sialic acid, 5 mM sialyllactose, anti-GM$_3$ antibody DH2, and anti-Gg3 MAb 2D4 are indicated on the ordinate. Values represent means of triplicate determinations.

Detailed Description of the Invention

As noted above, the present invention is directed toward methods for inhibiting cell-cell or cell-substratum (e.g., basement membrane) interactions by blocking carbohydrate-carbohydrate interactions. Carbohydrate-carbohydrate interactions may be important to at least two types of cell-cell and cell-substratum phenomena, namely, adhesion of a cell to another cell or substratum, and specific recognition between cells.

The process of cell adhesion consists of multiple steps. The initial step is specific recognition between two cells. The disclosure of the present invention shows that multivalent carbohydrate-carbohydrate interactions, mediated by bivalent cations, play a major role. Initial cell recognition is followed by nonspecific adhesion, primarily mediated by $Ca^{2+}$-sensitive adhesion molecules or by pericellular adhesive meshwork proteins consisting of fibronectin, laminin, and their receptor systems. The third step of cell adhesion is the establishment of intercellular junctions, e.g., "gap junctions," in which a cell-cell communication channel is opened through a specific structural protein.

The present invention also discloses that specific recognition between two cells is dependent upon the specific interaction between identical or nonidentical carbohydrate chains highly expressed in a cell and its counterpart. As disclosed within the present invention, agents that block specific carbohydrate-carbohydrate interactions (hereinafter referred to as "agents") will inhibit cell-cell and cell-substratum interactions. Suitable agents in this regard include saccharides, glycoconjugates and antibodies directed against carbohydrate determinants. The term "saccharides" includes oligosaccharides. The term "antibodies" refers to intact molecules, fragments thereof, or functional equivalents thereof, and may be genetically engineered. Examples of antibody fragments include F(ab')$_2$, Fab', Fab and Fv.

An example of a suitable glycoconjugate for use within the present invention is an oligosaccharide coupled to a peptide, such as lysyllysine. Such a glycoconjugate may be produced by coupling purified oligosaccharides to lysyllysine by reductive amination, e.g., as described by Schwartz and Gray (Arch. Biochem. Biophys. 181:542, 1977). This method is based on the ability of cyanoborohydride to reduce a Schiff base selectively at pH > 5. Briefly, unreduced oligosaccharide, L-lysyllysine 2HCl (Bachem Fine Chemicals, Torrance, Calif.) and sodium cyanoborohydride (Sigma Chemical Co., St. Louis, Mo.) are mixed in a molar ratio of 0.35:0.033:1.00 and dissolved in 0.2 M potassium phosphate (pH 8.0). After 72 hours at

$37^{\circ}$ C, the oligosaccharide-lysyllysine conjugate is separated from free oligosaccharide, cyanoborohydride, and buffer salts by P2 molecular sieve chromatography. Oligosaccharide-lysyllysine conjugates elute immediately after the void volume and are identified routinely in a spot test using 0.5% orcinol in a 10% $H_2SO_2$ spray.

For example, a glycoconjugate of lacto-N-fucopentaose (LFP) and lysyllysine may be prepared as follows. A mixture of LFP I (H hapten), II (Le$^a$ hapten), and II (X hapten) is obtained from human milk by the method of Kobata et al. (Meth. Enzymol. 50:216, 1978). LFP I, II, and III (unreduced) are separated after acetylation by preparative thin-layer chromatography on HPTLC plates (J. T. Baker Chemical Co., Phillisburg, N.J.) using a solvent system of butylacetate-acetone-water (25:8:1). After deacetylation, purified pentasaccharides are lyophilized and stored at $-20^{\circ}$ C. LFP III is coupled to the primary amino groups of lysyllysine by the procedure described above. The resulting LFP III-lysyllysine conjugate contains 3 moles of oligosaccharide per mole of lysyllysine.

As noted above, antibodies against carbohydrate determinants are suitable for use within the present invention. While polyclonal antibodies may be employed in the present invention, monoclonal antibodies (MAbs) are preferred. For embodiments of the present invention that pertain to cancer, suitable antibodies include those directed against tumor-associated carbohydrate antigens. Examples of tumor-associated carbohydrate antigens include Le$^X$-type, di-Le$^X$-type, sialylated Le$^X$-type, Le$^Y$-type, T, Tn, and sialyl-Tn-type antigens. MAbs may be produced against these antigens by immunization of mice with purified glycoplipid antigen coated on Salmonella minnesota (Young et al., J. Exp. Med. 150:1008-19, 1979). Suitable MAbs include SH-1, SH-2, 1B2, AH-6, FH-6, NBH-2, Cu-1, HH-8, TKH-1 and TKH-2. These MAbs are directed against the carbohydrate portion of tumor-associated glycolipids. SH-1, of the isotype IgG$_3$, recognizes the terminal five carbohydrate residues of Le$^X$, penta-Le$^X$. SH-2 is directed against dimeric Le$^X$, FH-6 against sialylated Le$^X$ (Fukushi et al., J. Biol. Chem. 259:10511-17, 1983), and AH-6 against Le$^Y$ (Abe et al., J. Biol. Chem. 258:11793-97, 1983). The MAb 1B2 reacts with the terminal Gal$\beta$1 → 4GlcNac of polylactosamine structures.

The procedure for the preparation of MAb SH-1 is representative. Lewis$^X$ antigen (III$^3$ Fuc nLc$^4$) is purified from a human liver tumor metastasized from colonic cancer. The tumor is homogenized three times in isopropanol:hexane:$H_2O$ and filtered. The organic extract is evaporated to dryness and partitioned three times by the method of Folch (J. Biol. Chem. 191:819, 1951). The combined upper phase is evaporated and dialyzed against distilled water in a spectrapore 3 (3500 mol. wt. cut-off) dialysis tubing. The dialysate is evaporated using excess ethanol and subjected to ion-exchange chromatography on DEAE-Sephadex A-25 column (4 x 50 cm). The sample is applied in chloroform:methanol:water (30:60:8). The nonbinding pass-through fraction is collected which contains Lewis$^X$ antigen. The sample is evaporated and subjected to high-pressure liquid chromatography (HPLC) on an iotrobead system consisting of isopropanol:hexane:water (55:40:5 to 55:25:20).

The fractions are pooled on the basis of staining with orcinol-sulfuric acid. The glycolipids migrating between standard H$_1$ and H$_2$ glycolipids are pooled and acetylated using pyridine-acetic anhydride. The acetylated glycolipids are applied to a preparative TLC plate and developed in dichloroethanol:acetone:water (40:60:0.03). Each isolated band is analyzed by NMR and permethylation analysis, and Le$^X$ band is collected.

Balb/c mice (female, 8-week-olds) are immunized with the purified Lewis$^X$ antigen (III$^3$ Fuc nLc4). The antigen (40 $\mu$g/100 $\mu$l ethanol) is injected into 800 $\mu$l phosphate-buffered saline (PBS) at $37^{\circ}$ C. The solution is further mixed with 250 $\mu$g of acid-treated S. minnesota(1 mg/ml PBS). The mixture is incubated at $37^{\circ}$ C for 30 min and lyophilized. The lyophilized powder is resuspended in 1 ml PBS. Mice are immunized every 10 to 14 days apart by tail vein injection of 250 $\mu$l of antigen suspension of PBS.

Three days after the last injection, animals are killed by cervical dislocation and spleens are aseptically excised. Lymphocytes are fused with mouse myeloma SP$^2$ cells (5:1) using polyethylene glycol. Clones are screened after 11 days of fusion. The clones at this stage are small. Clones were screened using a Pandex machine, in which antigen is coated on submicron polystyrene particles. Antigen-coated beads are mixed with the antibody supernatants, followed by the addition of FITC-goat anti-mouse IgG and IgM. This assay ("Pandex assay") is two- to fourfold more sensitive than traditional radioimmunoassay or ELISA assay and requires only 10 ng of antigen/well as opposed to 50 to 100 ng/well in other assays. This screening procedure further facilitates the selection of a high-affinity antibody to Lewis$^X$. The clones that test positive are cloned by single-cell dilution and are also tested by TLC immunostaining. Clones producing MAb SH-1, showing high reactivity in the Pandex assay and in TLC immunostaining, are selected and frozen.

The particular agent selected is determined by the structure of the specific carbohydrate chain that is to be blocked. The structure of carbohydrates bound to either lipids or proteins may be determined based on degradation, electron-impact direct-probe (EI) and field desorption (FD) mass spectrometry, and methylation analysis (techniques described, for example, in Nudelman et al., J. Biol. Chem. 261:5487-5495, 1986). Degradation analysis may be accomplished chemically and/or enzymatically, e.g., by glycosidases. The carbohydrate sequence suggested by degradation analysis may be assigned by methylation analysis (Hakomori, J. Biochem. 55:205-208, 1964) followed by chemical ionization mass spectrometry of permethylated sugars (Stellner et al., Arch. Biochem. Biophys. 155:464-472, 1973; Levery et al., Meth. Enzymol. 138:13-25, 1987). Alternatively, or in conjunction with these techniques, EI mass spectrometry may be performed on permethylated glycans or after appropriate degradation of intact glycans (Kannagi et al., J. Biol. Chem. 259:8444-8451, 1984; Nudelman et al., J. Biol. Chem. 263:13942-13951, 1988). Proton NMR spectroscopy of intact or methylated glycans may also be useful. Homogeneity of the carbohydrate sequence may be demonstrated based on various chemical and physical criteria, including FD mass spectrometry. Once the carbohydrate sequence has been determined, it will be evident to those of ordinary skill in the art to select an appropriate saccharide, glycoconjugate, or antibody against all or part of the sequence, as an agent for blocking the particular carbohydrate-carbohydrate interaction.

The inhibition of cell-cell or cell-substratum interactions has a variety of in vitro and in vivo uses. As noted above, the present invention provides a method for inhibiting cell-cell or cell-substratum interactions within a biological preparation. The method comprises incubating the biological preparation with an agent that blocks carbohydrate-carbohydrate interactions, thereby inhibiting cell-cell or cell-substratum interactions. Suitable biological preparations include cell cultures and biological fluids, such as serum, urine, synovial and cerebrospinal fluid. The present invention also provides a method for inhibiting cell-cell or cell-substratum interactions in a warm-blooded animal, such as man. The method comprises administering to a warm-blooded animal an effective amount of an agent that blocks carbohydrate-carbohydrate interactions, thereby inhibiting cell-cell or cell-substratum interactions. Suitable agents for either method include those described above. Saccharides and glycoconjugates will generally be incubated at a final concentration of about 0.5 to 10 mM or administrated at a concentration that achieves this concentration range in situ.

A representative example of a carbohydrate-carbohydrate interaction is the recognition of $Le^x$(Gal$\beta$1 → 4[Fuc$\alpha$1 → 3]GlcNac$\beta$1 → R) by $Le^x$ per se. $Le^x$ pentaosyl glycolipid (III$^3$FucnLc$_4$) was prepared from human colonic adenocarcinoma. Within the present invention, $Le^x$ is shown to be the structure recognizing $Le^x$. More specifically, liposomes containing $Le^x$ showed significant interaction with $Le^x$ glycolipid, but not other glycolipids, coated on a plastic surface. Second, liposomes containing $Le^x$ were observed to self-aggregate. Third, the interaction of $Le^x$ liposomes with immobilized $Le^x$ glycolipid and the self-aggregation of $Le^x$ liposomes were both specifically inhibited by LFP III. Fourth, the diffusability of tritium-labeled LFP III (from the lower to the upper Boyden chamber through a semipermeable membrane) was inhibited by $Le^x$ liposomes. In addition, the $Le^x$-$Le^x$ interaction was highly dependent on bivalent cations ($Ca^{2+}$, $Mg^{2+}$) and was inhibited by EDTA.

Another representative example of a carbohydrate-carbohydrate interaction is the specific interaction between gangliotriaosylceramide (Gg3; GalNAc$\beta$1 → 4Gal$\beta$1 → 4Glc$\beta$1 → Cer) and sialosyllactosylceramide (GM$_3$; NeuAc$\alpha$2 → 3Gal$\beta$1 → 4Glc$\beta$1 → Cer). Gg3 was prepared from guinea pig erythrocytes (Seyama and Yamakawa, J. Biochem. (Tokyo) 75:837-842, 1974) and GM$_3$ from dog erythrocytes (Klenk and Heuer, Z. Verdauungs u Stoffwechselkrankheiten 20:180-183, 1960). Within the present invention, Gg3 and GM$_3$ are shown to recognize one another. More specifically, liposomes containing GM$_3$ showed specific interaction with Gg3, but not other glycolipids, coated on a plastic surface. Pretreating the Gg3-coated solid phase with anti-Gg3 MAb inhibited the interaction with GM$_3$-containing liposomes. The Gg3/GM$_3$ interaction was promoted by bivalent cations ($Ca^{2+}$, $Mg^{2+}$) and was inhibited by EDTA.

As noted above, one aspect of the present invention provides a method for the inhibition of metastases in a warm-blooded animal. The method comprises administering to a warm-blooded animal a therapeutically effective amount of an agent that blocks carbohydrate-carbohydrate interactions, wherein at least one member of the carbohydrate-carbohydrate interaction resides on a malignant tumor cell. Sources of malignant tumor cells include melanoma, lung, breast, colorectal and urogenital cancers, such as bladder and prostate cancers. Suitable agents include those described above.

The formation of a secondary tumor colony at a distant site is a multistep process of which tumor invasion is the first step. Tumor cells locally invade host tissue barriers, such as the epithelial basement membrane, to reach the interstitial stroma, where they gain access to blood vessels (or lymphatic channels) for further dissemination. After invading the endothelial layer of the vessel wall, the circulating tumor cells are dislodged into the circulation and arrest in the precapillary venules of the target organ by adherence to endothelial cell lumenal surfaces, or exposed basement membranes. The tumor cells again invade the vascular wall to enter the organ parenchyma. Finally, the extravasated tumor cell must grow in a tissue different from where it originated.

Carbohydrate-carbohydrate interactions may be important to at least two steps in the formation of a metastasis. First, these interactions may be important when the circulating tumor cells arrest in the precapillary venules of the target organ by adherence to endothelial cell lumenal surfaces, or exposed basement membranes. Second, these interactions may be important to the process of cell adhesion between tumor cells growing in the target organ. By blocking these carbohydrate-carbohydrate interactions, metastases are effectively inhibited.

A representative example of the inhibition of tumor cell-tumor cell adhesion by the blocking of carbohydrate-carbohydrate interactions involves F9 teratocarcinoma cells. Within the present invention, the autoaggregation of F9 cells is shown to be inhibited by the Le$^x$ oligosaccharide, LFP III. More specifically, mouse teratocarcinoma F9 cells (Bernstine et al., Proc. Natl. Acad. Sci. USA 70:3899-3903, 1973) were grown on gelatin-coated plates in Dulbecco's modified Eagle's medium (DME) containing 4.5 g/l glucose and supplemented with 10% fetal calf serum (FCS) (v/v). The plates were prepared by incubating 0.01% gelatin in water for 30 min.

F9 cells grown to confluency on gelatin-coated plates were harvested using standard trypsin/EDTA solution and washed in DME containing 10% FCS. Next, the cells were carefully resuspended in a PBS solution (containing 0.9 mM $Ca^{2+}$, 0.5 mM $Mg^{2+}$ and 0.1 mM $Mn^{2+}$) and supplemented with 0.1% FCS. The cell samples were mixed carefully before pipetting, and both manual counting and counting with a Coulter Counter (Model ZBI, Coulter Electronics Inc., Hialeah, Fla.) were performed. Adjustments of the aperture on the Coulter Counter were made in order to correlate with the manual counting of the single cells, excluding aggregated cells. For aggregation assays, cells were adjusted to a concentration of about $2.6 \times 10^7$/ml. After 1 hr at 37°C, 50 µl aliquots were mixed with 50 µl of ethylenediaminetetraacetic acid (EDTA), LFP II, or LFP III solutions, and cells were incubated for an additional 15 min at 37°C. Final concentrations of EDTA, LFP II, and LFP III were 50 mM, 20 mM, and 2.0 mM, respectively. Before measurements on the Coulter Counter, the 100 µl volume sample was diluted to 10 ml and mixed carefully. In experiments were photomicrographs of cells were taken, approximately $0.8 \times 10^6$ cells were placed in different PBS solutions (final volume 100 µl) in small Petri dishes. The cell suspension was then covered by mineral oil and incubated at 37°C for 30-60 min, and the process of cell aggregation was observed under the microscope.

As shown in Figure 1, F9 cells aggregate in the presence of $Ca^{2+}$ and $Mg^{2+}$ (panel b); however, this self-aggregation was inhibited by EDTA (panel a) and by LFP III (panel d), but not by LFP II (panel c). The degree of F9 cell aggregation was semiquantitatively expressed by numbers of free (i.e., nonaggregated) cells (panel e), as determined by Coulter Counter with an appropriate aperture setting. Free cells increased greatly in the presence of LFP III, but much less in the presence of LFP II.

Another representative example of the inhibition of tumor cell-tumor cell adhesion by the blocking of carbohydrate-carbohydrate interactions involves melanoma cells. Within the present invention, the adhesion of a T cell lymphoma cell line to a melanoma cell line is shown to be inhibited by a variety of agents that block specific carbohydrate-carbohydrate interactions. In particular, the specific interaction of the mouse T cell lymphoma L5178 AA12 cell line (high expressor of Gg3) to mouse B16 melanoma cell line (high expressor of GM$_3$) was shown to be inhibited by such agents. As shown in Figure 2 (panels a and b), the mouse T cell lymphoma L5178 AV27 cell line, a genetically variant clone which does not express Gg3, does not show the specific interaction with B16 cells observed with AA12 cells.

Mouse B16 melanoma cells (which express a high level of GM$_3$) were purchased from American Type Culture Collection (Rockville, MD). Expression of GM$_3$ by B16 cells was defined by MAb DH2 (Dohi et al., Cancer Res. 48:5680-5685, 1988). Mouse T cell lymphoma line L5178 AA12 (which express a high level of Gg3, defined by MAb 2D4; Young et al., J. Exp. Med. 150:1008-1019, 1979) was cloned from 1C1 (Young et al., J. Immunol. 126:1-6, 1981). A variant clone, L5178 AV27, which does not express Gg3, was also described in Young et al., ibid. All cells were grown in RPMI 1640 containing 10% FCS.

B16 cells ($5 \times 10^4$/ml), highly expressing GM$_3$, were grown on 24-well plates (Falcon, Oxnard, CA) for 24 hrs in DME. The majority of adherent cells were washed with serum-free medium. AA12 cells (Gg3 expressors) or AV27 cells (Gg3 non-expressors) were grown in DME containing 2% FCS, 1 g/l glucose, and 50 µCi $^3$H-GlcNH$_2$ for 24 hrs. The labeled AA12 (71,000 cpm/$10^5$ cells) and AV27 (33,000 cpm/$10^5$ cells) were then collected by centrifugation, suspended in DME containing 2% FCS and 1 g/l glucose, and the cell population adjusted to $6 \times 10^5$/ml. This suspension (1 ml) was added to each well of a Falcon plate on which B16 melanoma cells were grown as adherent culture. The incubated plates were then washed 2x with serum-free fresh medium. L5178 lymphoma cells not adhering to B16 cells were washed out. The remaining cells in each plate were trypsinized, collected, and counted.

Figure 2 (panel c) shows the effects of 10 mM EDTA, 5 mM sialic acid, 5 mM sialyllactose, anti-GM$_3$ antibody DH2, and anti-Gg3 MAb 2D4 on adhesion of L5178 cells to B16 cells. B16 melanoma cells grown in 24-well plates and L5178 lymphoma cells were treated respectively with DH2 solution (2 µg/ml) and 2D4 solution (5 µg/ml), left for 30 min at room temperature, washed with serum-free fresh DME, and incubated with lymphoma cell suspension and melanoma cell layer for 5 hrs. Adhesion of AA12 cells on B16 melanoma was markedly inhibited by presence of sialyllactose, MAb DH2, or MAb 2D4. Adhesion of both AA12 and AV27 was greatly suppressed by EDTA.

For therapeutic uses, the agents of the present invention may be administered by a variety of ways, depending upon the type and location of the tumor to be treated. For example, administration can be intravenous, intraarterial, intraperitoneal, intrapleural, intrathecal, subcutaneous, by perfusion through a regional catheter, or by direct intralesional injection. Intravenous, intraarterial, or intrapleural administration is normally used for lung, breast, and leukemic tumors. Intraperitoneal administration is advised for ovarian tumors. Intraperitoneal administration is advised for brain tumors and leukemia. Subcutaneous administration is advised for Hodgkin's disease, lymphoma, and breast carcinoma. Catheter perfusion is useful for metastatic lung, breast, or germ cell carcinomas of the liver. Intralesional administration is useful for lung and breast lesions.

When administered to a warm-blooded animal (e.g., a human), the agent may be combined with water, an aqueous solution, or any physiologically acceptable carrier or diluent. Moreover, the agent may be administered in combination with an immunotherapeutic or chemotherapeutic agent. When a combination of agents is desired, each agent may be simultaneously administered or may be combined and administered as a single composition. The combination may be administered for therapeutic applications by a number of routes, including those described above.

The agents may be administered as a sterile aqueous solution in phosphate-buffered saline. The concentration of saccharide or glycoconjugate may be about 1-10 $\mu$g/ml. Dosage units of agent that achieve in situ concentrations of about 0.5-10 mM at the tumor site will be administered, normally daily for a period of several days. Diagnostic techniques, such as CAT scans, may be performed prior to and subsequent to treatment to confirm reduction in tumor volume. With those agents that are antibodies, it may be necessary to reduce the dosage and/or use antibodies from other species and/or hypoallergenic antibodies, e.g., hybrid human or primate antibodies, to reduce patient sensitivity.

It may be advantageous prior to initiating therapy to test a particular agent on a patient's tumor cells grown in vitro. For example, tumor cells removed from a patient during a biopsy may be cultured and then treated with a agent. Cell mortality and minimum dosages may be determined.

As noted above, another aspect of the present invention provides a method for inhibiting microbial infections in a warm-blooded animal. The method comprises administering to a warm-blooded animal a therapeutically effective amount of an agent that blocks carbohydrate-carbohydrate interactions, wherein at least one member of the carbohydrate-carbohydrate interaction resides on a microbe. Suitable agents include those described above. The term "microbe" includes bacteria, viruses, fungi and parasites.

Cell-cell and cell-substratum interactions may play at least two roles in microbial infections, namely, in the adherence of a microbe to a host surface and in the recognition of microbial cells in adherence and colonization. The first step in the process of colonization by a microorganism such as bacteria is the adherence to a host surface. Vulnerable membranes include intestinal, urogenital, and bronchial epithelia and hard surfaces, such as dentin. Microorganisms also possess the ability to bind platelets and fibrin, thereby permitting their deposition on heart valves, which results in endocarditis. Carbohydrate-carbohydrate interactions are important to the adherence of invading microorganisms to host surfaces. By blocking these interactions, potential microbial invaders are prevented from gaining a foothold in a mammalian host.

Another way that microorganisms adhere to a host is via another bacterial cell that is already attached to a host surface. This phenomenon is termed "coaggregation" and is defined as the recognition between surface molecules on two different bacterial cell types so that a mixed cell aggregate is formed. These interactions play a major role, for example, in the adherence of human oral bacteria. It is well known that within minutes after teeth are professionally cleaned, the enamel surface is repopulated with bacteria. Bacteria must attach or they will be washed away by normal salivary flow. Consequently, when all the sites on the tooth are filled, the incoming bacteria must recognize and adhere to already attached bacteria.

The interactions between different bacteria are highly specific in that only certain cell types are partners. Although coaggregation has been thought to be mediated by specific recognition between complementary lectin-carbohydrate molecules on the participating partners, coaggregation is another example within the present invention of a specific carbohydrate-carbohydrate interaction.

This method for inhibiting microbial infections is useful for prophylactic and therapeutic treatment of infectious diseases induced by a variety of microorganisms, including bacterial, viruses, fungi and parasites. The agents may be used either alone, or in combination with a known antimicrobial agent, for prophylactic and therapeutic antimicrobial applications. Additionally, they may be administered in combination with chemotherapy.

Use of a combination of an antimicrobial agent together with an agent suitable for use in the present invention is particularly advantageous when the antimicrobial agent has toxic side effects. For example, antibacterial aminoglycosides such as gentamicin, streptomycin, and the like are known to have serious toxicity, particularly ototoxicity and nephrotoxicity, which reduce the usefulness of such antimicrobial agents (See Goodman and Gilman's The Pharmacological Basis of Therapeutics, 6th ed., A. Goodman Gilman et al. (eds.); Macmillan Publishing Co., Inc., New York, pp. 1169-71 (1980)). Use of an agent in combination with such agents permits a lower dosage of the toxic antimicrobial agents while still achieving therapeutic effectiveness.

The agents of the present invention may be subjected to standard tests in order to determine the minimum inhibitory concentration needed to inhibit a range of bacterial microorganisms in vitro.

Acute toxicity may be evaluated following a single intravenous injection of agent at a variety of doses into normal animals. Treated animals are then observed for 30 days post-injection. Chronic toxicity may be evaluated, for example, up to 60 days following twice weekly intravenous injections of saline solution containing agent. Body and organ weights, gross and microscopic pathology, serum electrolytes, solutes and serum enzymes indicative or renal and hepatic function are monitored. The serum components include glucose, blood urea nitrogen (BUN), uric acid, cholesterol, triglycerides, total protein, albumin, globulin, creatinine, calcium, phosphorous, sodium, potassium, chloride, bicarbonate and anion gap. The enzymes include: alkaline phosphatase, lactic dehydrogenase, serum glutamic oxalacetic transaminase, serum glutamic pyruvic transaminase and creatinine phosphokinase.

Acute and chronic pyrogenicity may also be evaluated. For example, animals receive a single or multiple injections of increasing doses of an agent over a three-hour period. Body temperature is determined at 15-minute intervals following bolus injections.

The agents may be administered for prophylactic and therapeutic applications by a number of routes, including: orally, by injection (including intradermally, intravenously, intraperitoneally, subcutaneously, intramuscularly, etc.), topically as a dry powder or in a suitable physiologic carrier, by topical application to nasal and nasopharyngeal linings, and by inhalation via aerosolization and application to respiratory tract linings, etc. In addition, the agent may be impregnated on bandages, sutures or dressings either as a dry powder or in a suitable physiologic carrier. Saccharides and glycoconjugates will generally be administered at a concentration that achieves an in situ concentration of about 0.5-10 mM at the infection site.

When administered to a warm-blooded animal (e.g., a human), the agent may be combined with water, an aqueous solution or any physiologically acceptable pharmaceutical carrier or diluent. Moreover, as described above, the agent may be administered in combination with an antimicrobial agent. When a combination of an agent and an antimicrobial agent is desired, each agent may be simultaneously administered or may be combined with one or more antimicrobial agents and administered as a single composition. The combination may be administered for prophylactic and therapeutic applications by a number of routes, including those described above. This discussion, regarding a combination of one or more agents and one or more antimicrobial agents, applies to combinations which include chemotherapeutic agents.

Within a related aspect, the present invention provides a method for inhibiting dental plaque in a warm-blooded animal. The method comprises administering to a warm-blooded animal an effective amount of an agent that blocks carbohydrate-carbohydrate interactions, wherein at least one member of the carbohydrate-carbohydrate interaction resides on an oral bacterial cell. Suitable agents include those described above.

Inhibition of plaque formation by an agent may be tested using extracted teeth. For example, freshly extracted human teeth are collected in tap water and scalded to remove deposits. They are then autoclaved in test tubes for 15 minutes at 120°C. After sterilization, the teeth are immersed for 2 minutes in 2 ml of test suspensions containing agents. They are taken out and air dried for 1 minute and washed with 200 ml distilled water. These treated teeth are suspended in 10 ml of thioglycollate medium and inoculated with 0.1 ml of $10^{-2}$ dilution of 48-hour-old Streptococcus mutans culture. Teeth processed as above without the agent treatment serve as the control.

A toothpaste containing one or more agents may be prepared. In addition to the agent, a toothpaste typically includes glycerin, saccharin, carboxymethyl-cellulose, water, silica dioxide, and a flavoring additive. The saccharides and glycoconjugates will generally be present at a concentration of 1-50 mM.

As noted above, in another aspect of the present invention provides a method for the inhibition of fertilization in a warm-blooded animal (e.g., a human). The method comprises administering to the animal an effective amount of a composition comprising an agent that blocks carbohydrate-carbohydrate interactions between sperm and egg, and a physiologically acceptable carrier or diluent. Suitable agents include those described above.

Prior to interacting with the plasma membrane of an unfertilized egg, a capacitated spermatozoon must penetrate the zona pellucida. A prerequisite for penetration is interaction of the spermatozoon with this extracellular coat, which leads to the state of adhesion called "binding." At least one glycoprotein in the zona pellucida has been implicated as a sperm receptor. The identity of the egg binding molecule on sperm, however, has been unclear. The literature (e.g., Shur, J. Biol. Chem. 257:6871-6878, 1982) focuses on enzymes, such as glycosyltransferases and proteinases, and lectin-like proteins. The present invention, however, is based upon carbohydrate-carbohydrate, rather than carbohydrate-amino acid, interactions. By blocking the carbohydrate-carbohydrate interactions between sperm and egg, the sperm is unable to adhere to the zona pellucida and fertilization is inhibited.

The contraceptive compositions of the present invention will generally be in the form of a gel, foam, cream, or suppository which is suitable for intravaginal use. Although these compositions may be used alone, it is generally preferred that they be used in combination with a barrier method of contraception in order to realize their maximum effectiveness. For example, a contraceptive composition in the form of a gel is applied to a diaphragm in an amount of about 1 ml to 20 ml, preferably about 10 ml, and the diaphragm is inserted prior to coitus.

As noted above, the contraceptive compositions will include an agent that blocks carbohydrate-carbohydrate interactions between sperm and egg, in combination with a physiologically acceptable carrier or diluent, generally a water-soluble compound. Preferred carriers include polyethylene glycol and polyoxyethylene-polyoxypropylene block copolymers ("poloxamers"). The use of poloxamers in intravaginal contraceptive compositions is disclosed by Vickery et al. (U.S. Patent No. 4,368,186, herein incorporated by reference). The compositions may further contain additional ingredients, including buffers, antimicrobial agents, or glycerol, which may be added to control viscosity. Water may also be added. Agents are included in a concentration suitable to provide the contraceptive effect, generally the saccharides and glycoconjugates will be added at a concentration of about 1-50 mM.

Furthermore, the agents may be used in combination with other proteins and/or small molecules that inhibit other factors in the fertilization pathway. Accessory molecules suitable for use within the present invention include benzamidine, 4-aminobenzamidine, 4'-nitrophenyl 4-guanidinobenzoate and others (Beyler and Zaneveld, J. Reprod. Fertil. 66:425-431, 1982). The contraceptive compositions may also include inhibitors of hyaluronidase, beta-glucuronidase, and beta-N-acetylglucosaminidase (Joyce et al., Biology of Reproduction 35:336-346, 1986). Contraceptive compositions will be formulated to provide maximal contraceptive effect combined with minimal hazard of toxicity and irritancy.

In addition to the compositions described above, the agents described herein may be substituted for other spermicidal agents in devices and compositions known in the art. For example, these proteins may be used in the suppositories disclosed by Kazmiroski et al. (U.S. Patent No. 4,384,003) or in the disposable diaphragm of Dunn et al. (U.S. Patent No. 4,589,880).

The ability of an agent to inhibit fertilization in vitro may be tested using sperm and ova obtained (as described below) from the hybrid mouse strain B6D2F1. This strain is chosen because a 75% to 85% in vitro fertilization rate is routine and the female mice respond well to superovulatory drugs, producing an average of 30 normal oocytes per animal.

Oocytes are collected from 3- to 4-week-old female B6D2F1 mice. The animals receive 5 international units of pregnant mare's serum gonadotropin to initiate follicular growth. After 48 hours, 5 international units of human chorionic gonadotropin are given to effect ovulation. Twelve hours after the last injection, oviducts are removed and oocytes are collected by puncture of the ampullary region, allowing the release of cumulus enclosed oocytes (cumulus clots) into 0.2 ml Fraser's medium (Fraser and Drury, Biol. Reprod. 13:513-518, 1975) under paraffin oil. To randomize oocyte distribution, all cumulus clots are collected into one dish of medium and then placed into the fertilization medium. Oocytes are collected, on average, 15 minutes before sperm addition.

Sperm are collected from the cauda epididymus of 9- to 14-week-old male B6D2F1 mice to proven fertility. Each epididymus contains approximately 40 million sperm with 70% to 80% motility. The cells from one epididymus are placed into 0.2 ml Fraser's medium (agents at varying concentrations) under paraffin oil and incubated for one hour at 37°C in a humidified 5% $CO_2$ in air atmosphere.

After the one-hour capacitation period, the sperm are counted by hemocytometer and 106 sperm cells/ml are added to 0.2 ml Fraser's medium (free of or containing agent) containing cumulus cell-enclosed oocytes. The sperm/oocyte mixture is incubated at 37°C in a 5% $CO_2$ in air atmosphere for two hours, after which the oocytes are removed and washed three times to remove excess spermatozoa. The washed oocytes are placed into individual 0.01 ml drops of medium under paraffin oil and return to the 37°C environment. After an additional three hours of incubation, the oocytes are observed for signs of fertilization (the presence of both the second polar body and two pronuclei). To confirm fertilization, the oocytes are incubated an additional 24 hours and observed for the formation of two-cell embryos. The above experiments are designed to analyze the effective inhibitory concentration of an agent.

For determining the effectiveness of the agents in vivo, a cream is prepared by dissolving the agent in 5 ml of phosphate-buffered saline (PBS). This solution is emulsified with 15 ml of K-Y Jelly (Johnson & Johnson). The cream (100 μl) is administered intravaginally to female mice prior to copulation. Control females are untreated or received a control cream consisting of 5 ml of PBS plus 15 ml of K-Y Jelly. Mice are sacrificed 12 days after copulation and the number of fetuses is noted.

Yet another aspect of the present invention provides a method for inhibiting interaction of a plant with a pathogen, such as bacteria, viruses, nematodes, and fungi. The method comprises administering to the plant an effective amount of an agent that blocks carbohydrate-carbohydrate interactions, wherein at least one member of the carbohydrate-carbohydrate interaction resides on a plant pathogen.

Cell-cell and cell-substratum interactions are important in at least two aspects of plant infections, namely, attachment of the pathogen to the plant and recognition of the pathogen during colonization. One of the earliest stages in the interaction between a plant pathogen, e.g., Agrobacterium, is the attachment of the bacteria to the plant cell surface. The initial attachment of single bacterial cells is followed by massive aggregation of the bacteria at the plant cell surface. Attachment is important for virulence, as demonstrated by the fact that Agrobacterium mutants that are defective in attachment are either avirulent or severely attenuated in virulence.

The agents of this invention are useful for inhibiting infection of plants by phytopathogens, such as bacteria and fungi. In agriculture, severe problems are faced in the raising of cotton, beans, corn and other crops because of the loss of yield per acre due to the action of soilborne fungi on seed and on the roots of the young plants. Control or elimination of these losses can be accomplished by the use of the agents described herein as soil disinfectants in accordance with the invention. They can also be used for the control of bacterial and fungal diseases on trees and stored crops.

The agents may, if desired, be applied in combination with other agricultural chemicals, such as insecticides, acaricides, nematocides, fungicides, antiviral agents, herbicides and plant-growth regulators, as well as with fertilizers. Various adjuvants that are known in the art may also be employed.

Typical formulations for use in the method of the invention include the agent in combination with an agriculturally acceptable carrier or extender, preferably with a surface active agent, and optionally with other active ingredients. Suitable formulations include granules of various sizes, dusts, wettable powders, emulsifiable concentrates, solutions, dispersions, and the like, with the choice depending upon the environment factors at the situs of infestation and the method of application selected. A typical formulation may vary widely in concentration of the active ingredient depending upon the additives and carriers used and the desired mode of application. Thus, active ingredient may be present at a concentration of 0.1% to 99.5% by weight of the formulation. An agriculturally acceptable carrier may comprise about 99.5% by weight to as low as 0.5% by weight of the formulation. Compatible surface active agents, if employed in the formulation, may be present at various concentrations, suitably in the range of 1% to 30% by weight of the formulation.

The formulation may be used as such or diluted to a desired use dilution with a diluent or carrier suitable for facilitating dispersion of the active ingredient. The concentration of the active ingredient may be in the range of about 0.01% to about 10% by weight.

In the formulations mentioned above, as those skilled in the art will appreciate, a wide choice of diluents, emulsifiers, wetting agents or solvents, is available. The particular diluent, emulsifier, solvent or wetting agent best suited for a given formulation is readily determined by one skilled in the art.

The phytotoxicity of an agent may be determined in post-emergence treatment essentially as described in U.S. Patent No. 4,460,709. For example, plastic trays (35 x 25 x 10 cm (in depth)) are filled with soil, and seeds are sowed in the trays and grown for 2 to 3 weeks in a greenhouse. A required amount of the agent to be tested is sprayed on the foliage of the test plants over the top by means of a small hand sprayer. After this foliar application, the plants are grown for an additional 3 weeks in the greenhouse. The activity is then examined. At the above-described foliar application, the test agents are applied at a spray volume of 5 liters per acre with addition of a wetting agent. The height of the plants at the foliar application varies depending upon the species, but they are at the 2- to 4-leaf state and 2 to 10 cm high. At this foliar application, corn is in the 2-leaf stage, wheat in the 2-leaf stage, rice plant in the 3-leaf stage, cotton in the 1-leaf state, soybean in the second trifoliate stage and sugarbeet in the 2-leaf stage.

The application of the agents as antifungal agents may be compared to a commercially available fungicide, such as that known under the generic name "trisorine." Wheats (var.: Nohrin No. 61) are grown up to the one-leaf stage in a flower pot of 9 cm in diameter. Then each of the test compounds is diluted with water and sprayed on the test plants in a rate of 15 ml/pot. The test plants are placed in a chamber kept at 20° C. The plants are then inoculated with Puccinia recondita and placed under a humid chamber for 18 hours, incubated in a 20° C chamber, and grown under a fluorescent lamp for an additional 10 days.

The infection state is observed, and the disease severity is calculated on the basis of the following standard:

| Disease Index | Infection State |
|---|---|
| 0 | No infectious spot in the examined leaf |
| 1 | Less than 10 infectious spots on the examined leaf |
| 2 | 11-20 infectious spots on the examined leaf |
| 4 | 21-50 infectious spots on the examined leaf |
| 8 | More than 51 infectious spots on the examined leaf |

Observation of less severe symptoms in plants pretreated with the agents (as compared with plants sprayed with a reagent blank) confirms the protective effect of the agents.

The ready-to-use preparations (which may be prepared from suitable formulations by, for instance, dilution with water) may be applied in any usual manner, for instance, by spraying, such as liquid spraying, misting, atomizing, dusting, scattering, watering, fumigating, by soil application, such as mixing, painting, banding and dressing (dust-coating), or by immersion. For use, the agents described herein can be applied neat or employed in a diluted form. Satisfactory diluents include any inert material not destructive of the antimicrobial activity and especially liquid formulations comprising aqueous dispersions, solutions, and emulsions. Solid diluents include talc, corn starch, alumina and diatomaceous earth. The antimicrobial agents of this invention can also be deposed on materials such as natural fibers, including paper, cotton, wool and synthetic fibers, such as nylon, polypropylene, as well as upon inanimate surfaces, including hard surfaces such as wood, glass, metal, tile, rubber, plastic, and porous surfaces such as concrete, leather and the like.

Within this aspect of the invention, the agent may be applied by incorporating a formulation thereof into the soil in which agricultural crops have been or will be planted. This may be achieved by broadcasting the formulation over the planted area or the area to be planted or be banding the application in the root zone where plants have been or are to be planted. It will be readily apparent where the latter method is employed that an amount of formulation sufficient to supply an effective concentration of the agent in the soil must be applied to the root zone.

Wettable powder formulations for use as a dispersant in water represent a practical means for good distribution in soil. Other methods of achieving the same results include the preparation of dusts. Some of the agents useful in this invention can be blended as fine powders with the commonly used powder diluents, such as talc, clay-refined silicates, wood flour, sand, magnesium oxide, calcium carbonate, fuller's earth, kaolin, diatomaceous earth, mica, pumice and the like. The mixtures may be finely powdered, e.g., to the 1-10 micron average particle size, or may be made by blending the already finely powdered ingredients. The wettable powders can be prepared by the addition of 0.15% of a wetting agent to the powder blends.

For agronomic applications, the dusts may be applied to the seed surrounding soil at the time of planting. The concentration of the agent is adjusted to give an effective, nonphytotoxic dosage in the soil. For most economical and effective use the dusts can be applied in bands of 6 to 8 inches centered on the rows just prior to seeding. The material can then be rototilled to a depth of several inches. This mode of treatment saves material and protects the root system of young plants against microbial attack. For the protection of a given crop, such as cabbage, the band spread of antimicrobial can vary from 8 inches for black root disease to 12-15 inches for club root disease prevention. Similarly, the depth to which the fungicide should be distributed can vary from 2 to 6 inches. Optimum placement of the agents will depend on the particular plant, and the skilled artisan will choose those conditions appropriate for the plant to be protected. Inclusion of the agent with a band of fertilizer may yield the desired results as one of ordinary skill in the art will understand.

Many dispersing compounds are commercially available which are nonphytotoxic at the required concentrations. These may, for example, be alkali metal and amine salts of sulfated and sulfonated acids, alcohols and oils, or polyethoxylated alkyl phenols, long chain fatty amine quaternary salts, partial phenols, partial fatty acid esters of polyhydric alcohols, etc. Many of these dispersing compounds are available in solvent-soluble form. The manner of application to the soil is similar to the dusts. Spray equipment is used to spread the suspensions or emulsions over the soil and by discing, the agents can be uniformly distributed to varying depths. Spray application is also effective for band-limiting the dosages.

Other agricultural uses for these formulations are by application to the phytopathogen involved foliar surface areas. Some of the diseases which are of commercial importance because they decrease yield and quality are fire blight of apple and pear, bacterial spot on stone fruit, cheery leaf spot, walnut blight, common blight of bean, bacterial spot of tomato and pepper, and potato seed piece decay. The agents may be active against diseases caused by the genera Agrobacterium, Pseudomonas, Xanthomonas, Erwinia, and Corynebacterium. They may be applied as dusts, powder dispersions in water, as emulsions in water, or as aqueous dipping baths. Other plant diseases which may be controlled by treatment with these formulations are fungal in origin, such as the many kinds of powdery mildew and leaf scabs.

In addition to the interaction of pathogens with plants, beneficial plant symbionts, such as Rhizobium species, also attach to plant cell surface. Rhizobium strains have well-defined and generally narrow host ranges involving one or a few plant genera, usually but not always plant legumes. Both the bacteria and the plant contribute to the specificity of the symbiosis. Although lectins have been suggested to be the molecule responsible for joining the two symbiotic partners, this is another example within the present invention of where carbohydrate-carbohydrate interactions provide the specificity in the cell-cell recognition process. Agents of the general types described above may, if desired, be selected to inhibit plant symbionts.

From the foregoing, it will be evident that, although specific embodiments of the invention have been described herein for purposes of illustration, various modifications may be made without deviating from the spirit and scope of the invention.

The features disclosed in the foregoing description, in the claims and/or in the accompanying drawings may, both, separately and in any combination thereof, be material for realising the invention in diverse forms thereof.

## Claims

1. An agent that blocks carbohydrate-carbohydrate interactions for use within a method for inhibiting cell-cell or cell-substratum interactions in a warm-blooded animal.

2. An agent that blocks carbohydrate-carbohydrate interactions, wherein at least one member of the carbohydrate-carbohydrate interaction resides on a malignant tumor cell, for use within a method for inhibiting metastases in a warm-blooded animal.

3. The agent of claim 2 wherein the malignant tumor cell is a melanoma, lung, breast, colorectal, or urogenital cancer cell.

4. An agent that blocks carbohydrate-carbohydrate interactions, wherein at least one member of the carbohydrate-carbohydrate interaction resides on a microbe, for use within a method for inhibiting microbial infections in a warm-blooded animal.

5. The agent of claim 4 wherein the microbe is selected from the group consisting of bacteria, viruses, fungi, and parasites.

6. An agent that blocks carbohydrate-carbohydrate interactions, wherein at least one member of the carbohydrate-carbohydrate interaction resides on an oral bacterial cell, for use within a method for inhibiting dental plaque in a warm-blooded animal.

7. The agent of claim 6 wherein the oral bacterial cell is a Streptococcus mutans.

8. A composition comprising an agent that blocks carbohydrate-carbohydrate interactions between sperm and egg, and a physiologically acceptable carrier or diluent, for use within a method for inhibiting fertilization in a warm-blooded animal.

9. The composition of claim 8 wherein the composition is in the form of a gel, foam, cream, or suppository suitable for intravaginal use.

10. The composition of claim 8 wherein the carrier or diluent is polyethylene glycol or a polyoxyethylene-polyoxypropylene block copolymer.

11. The composition of claim 8 wherein the agent is a saccharide, a glycoconjugate or an antibody directed against a carbohydrate determinant.

12. An agent that blocks carbohydrate-carbohydrate interactions, wherein at least one member of the carbohydrate-carbohydrate interaction resides on a plant pathogen, for use within a method for inhibiting plant-plant pathogen interactions.

13. The agent of claim 12 wherein the plant pathogen is selected from the group consisting of Agrobacterium, Pseudomonas, Xanthomonas, Erwinia, and Corynebacterium.

14. A method for inhibiting cell-cell or cell-substratum interactions within a biological preparation, comprising:

incubating the biological preparation with an agent that blocks carbohydrate-carbohydrate interactions, thereby inhibiting cell-cell or cell-substratum interactions.

15. The method of claim 14 wherein the biological preparation is a cell culture or a biological fluid.

16. The method of claim 14 wherein the agent is a saccharide, a glycoconjugate or an antibody directed against a carbohydrate determinant.

17. The agent of claims 1, 2, 4, 6, or 12 wherein the agent is a saccharide, a glycoconjugate or an antibody directed against a carbohydrate determinant.

# FIGURE 1

# FIGURE 2